(19) Europäisches Patentamt
European Patent Office
Office européen des brevets

(11) **EP 4 338 684 A1**

(12) **EUROPEAN PATENT APPLICATION**
published in accordance with Art. 153(4) EPC

(43) Date of publication:
**20.03.2024 Bulletin 2024/12**

(21) Application number: **22807107.2**

(22) Date of filing: **03.03.2022**

(51) International Patent Classification (IPC):
**A61B 8/14** *(2006.01)*

(52) Cooperative Patent Classification (CPC):
**A61B 8/14**

(86) International application number:
**PCT/JP2022/009077**

(87) International publication number:
**WO 2022/239400 (17.11.2022 Gazette 2022/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.05.2021 JP 2021080323**

(71) Applicant: **Furuno Electric Co., Ltd.
Nishinomiya-City, Hyogo 662-8580 (JP)**

(72) Inventor: **ISERI, Kensuke
Nishinomiya-City, Hyogo 662-8580 (JP)**

(74) Representative: **Müller Hoffmann & Partner
Patentanwälte mbB
St.-Martin-Strasse 58
81541 München (DE)**

(54) **ULTRASONIC IMAGING DEVICE, ULTRASONIC IMAGING METHOD, ULTRASONIC IMAGING SYSTEM, AND ULTRASONIC IMAGING PROGRAM**

(57) [Problem] To provide an ultrasonic imaging device that generates a clear composite image of a transverse section of a specimen. [Solution] An ultrasonic imaging device 3 comprises: an ultrasonic receiver (351) that receives, via a probe disposed on the surface of a specimen, ultrasonic waves which are transmitted in two scan modes, namely, a first scan mode, and a second scan mode having a wider imaging range than the first scan mode, the ultrasonic waves being transmitted from the probe toward the interior of the specimen and being reflected in the interior of the specimen; a first fragment image generator (352) for generating, on the basis of the ultrasound waves, a first fragment image obtained by partially imaging the interior of the specimen in the first scan mode; a second fragment image generator (353) for generating, on the basis of the ultrasound waves, a second fragment image obtained by partially imaging the interior of the specimen in the second scan mode; and a cross-sectional image synthesizer (354) that generates a composite image of a cross-section of the specimen by non-uniformly synthesizing the first fragment image and the second fragment image.

FIG. 2

## Description

Technical Field

The present invention relates to an ultrasonic imaging device, an ultrasonic imaging method, an ultrasonic imaging system, and an ultrasonic imaging program for imaging the interior of a specimen using ultrasonic waves.

Background

The quadriceps femoris muscle is the muscle of the thigh and controls movements such as pulling up the thigh and extending the knee joint. As the muscle mass of the quadriceps femoris muscle decreases significantly with aging, the decrease in the quadriceps femoris muscle is a factor in walking difficulties and falls for elderly people. Therefore, walking difficulties and falls of elderly people have been diagnosed by ascertaining the muscle mass of the quadriceps femoris muscle or the like. For example, a CT (Computed Tomography) device or an MRI (Magnetic Resonance Imaging) device has been used to image the entire transverse section of the thigh including the quadriceps femoris muscle.

However, a CT device or an MRI device is expensive and requires a longer time for imaging, so a simpler imaging technique is desired. Thus, various techniques have been devised for imaging a wide range of the transverse section of a human body using ultrasonic waves.

For example, Patent Literature 1 discloses a technique for imaging the transverse sections of the thigh, upper arm, abdomen, etc. of a human body, which is a specimen, using a probe that transmits and receives ultrasonic waves. According to the technique of Patent Literature 1, an operator moves the probe along a transverse section around the target to be imaged and continuously captures images while keeping the probe at an appropriate angle with respect to the surface of the human body, and the captured ultrasonic images are synthesized so as to acquire a panorama composite image (hereinafter also simply referred to as "composite image") of a wide range of the transverse section with respect to a portion of the target.

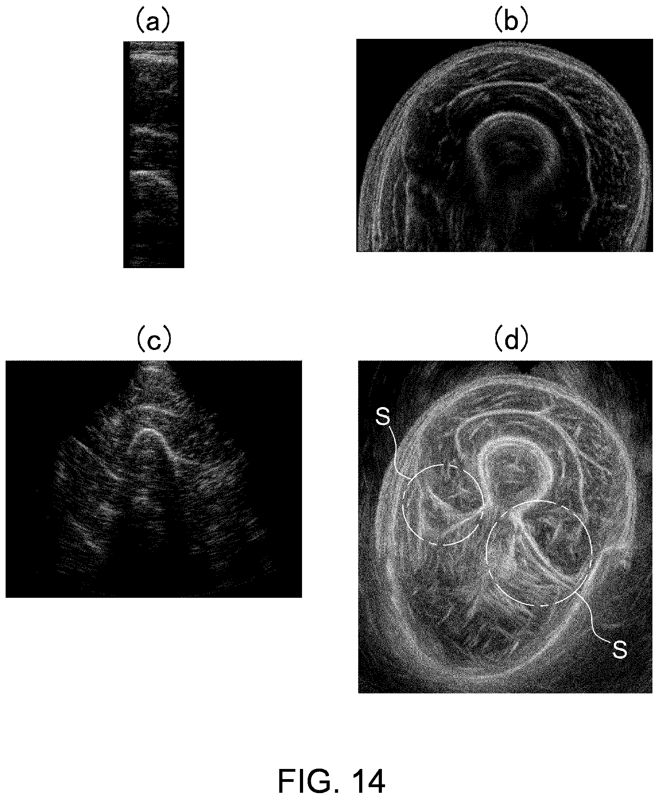

FIG. 14 is the ultrasonic images according to the conventional art, obtained by imaging a transverse section of the thigh in a linear scan mode and a sector scan mode, respectively.

When an examination is performed using ultrasonic imaging, the mode is switched between a plurality of ultrasonic scan modes according to the portion of the specimen. The ultrasonic scan modes include, for example, a linear scan mode, a sector scan mode, a convex scan mode, etc.

In the linear scan mode, as exemplified in (a) of FIG. 14, a range that extends in a band shape from the ultrasonic transmission and reception surface of the probe can be clearly imaged. The linear scan mode is mainly used for examination of tissue located in a portion near the body surface of the human body. If the periphery of the thigh is continuously imaged only in the linear scan mode, a panorama composite image of a transverse section as exemplified in (b) of FIG. 14 is acquired.

In the sector scan mode, as exemplified in (c) of FIG. 14, a range that expands in a fan shape, with a wider angular range than the range that extends in a band shape from the ultrasonic transmission and reception surface of the probe, can be imaged. The sector scan mode is mainly used for examination of tissue over a wide range, including a portion far from the body surface of the human body. If the periphery of the thigh is continuously imaged only in the sector scan mode, a panorama composite image of a transverse section as exemplified in (d) of FIG. 14 is acquired.

Citation List

Patent Literature

[Patent Literature 1] International Publication No. 2017/010193

SUMMARY OF INVENTION

Technical Problem

In the composite image of the transverse section of the thigh obtained only in the sector scan mode shown in (d) of FIG. 14, the entire transverse section of the thigh and the septum between muscles (region S surrounded by a one-dot chain line) are clearly imaged, but noise appears inside the muscle and the image is not clear. In contrast, in the composite image of the transverse section of the thigh obtained only in the linear scan mode shown in (b) of FIG. 14, the interior of the muscle is clearly imaged, but the imaging range is narrow and the septum is not imaged. This is because, if the contour portion of the internal tissue of the object to be imaged extends vertically from the surface of the object to be imaged, it is not possible to clearly receive the ultrasonic waves reflected by the contour portion in the linear scan mode. As described above, the composite image obtained only in the linear scan mode and the composite image obtained only in the sector scan mode both have merits and demerits.

The purpose of the present invention is to provide an ultrasonic imaging device that generates a clear composite image of a transverse section of a specimen.

Solution to Problem

An ultrasonic imaging device according to the present invention includes: an ultrasonic receiving unit configured to receive ultrasonic waves that are transmitted in two scan modes, which are a first scan mode and a second scan mode having a wider imaging range than the first scan mode, from a probe arranged on a surface of a specimen toward an interior of the specimen and that are reflected in the interior of the specimen with the probe; a first fragment image generating unit configured to generate a first fragment image obtained by partially imaging the interior of the specimen in the first scan mode based on the ultrasonic waves; a second fragment image generating unit configured to generate a second fragment image obtained by partially imaging the interior of the specimen in the second scan mode based on the ultrasonic waves; and a cross-sectional image synthesizing unit configured to generate a composite image of a cross section of the specimen by non-uniformly synthesizing the first fragment image and the second fragment image.

An ultrasonic imaging method according to the present invention includes: an ultrasonic reception step of receiving ultrasonic waves that are transmitted in two scan modes, which are a first scan mode and a second scan mode having a wider imaging range than the first scan mode, from a probe arranged on a surface of a specimen toward an interior of the specimen and that are reflected in the interior of the specimen with the probe; a first fragment image generation step of generating a first fragment image obtained by partially imaging the interior of the specimen in the first scan mode based on the ultrasonic waves; a second fragment image generation step of generating a second fragment image obtained by partially imaging the interior of the specimen in the second scan mode based on the ultrasonic waves; and a cross-sectional image synthesizing step of generating a composite image of a cross section of the specimen by non-uniformly synthesizing the first fragment image and the second fragment image.

An ultrasonic imaging system according to the present invention includes: a probe configured to transmit ultrasonic waves from a surface toward an interior of a specimen and receive the ultrasonic waves that are reflected in the interior of the specimen; and the ultrasonic imaging device according to the present invention.

An ultrasonic imaging program according to the present invention enables a computer to operate as: an ultrasonic receiving unit configured to receive ultrasonic waves that are transmitted in two scan modes, which are a first scan mode and a second scan mode having a wider imaging range than the first scan mode, from a probe arranged on a surface of a specimen toward an interior of the specimen and that are reflected in the interior of the specimen with the probe; a first fragment image generating unit configured to generate a first fragment image obtained by partially imaging the interior of the specimen in the first scan mode based on the ultrasonic waves; a second fragment image generating unit configured to generate a second fragment image obtained by partially imaging the interior of the specimen in the second scan mode based on the ultrasonic waves; and a cross-sectional image synthesizing unit configured to generate a composite image of a cross section of the specimen by non-uniformly synthesizing the first fragment image and the second fragment image.

Effects of Invention

According to the present invention, an ultrasonic imaging device can be provided for generating a clear composite image of a transverse section of a specimen.

BRIEF DESCRIPTION OF DRAWINGS

FIG. 1 is a schematic diagram showing the configuration of the ultrasonic imaging system 1.
FIG. 2 is a block diagram showing the configuration of the ultrasonic imaging device 3.
FIG. 3 is a schematic diagram for illustrating the method of synthesizing different types of fragment images, performed by the ultrasonic imaging device according to the first embodiment.
FIG. 4 is a flowchart showing the processing procedure of the ultrasonic imaging method according to the first embodiment.
FIG. 5 is a flowchart showing the detailed processing procedure of step S6A in the first embodiment.
FIG. 6 is an example of the panorama composite image of the transverse section of the thigh, generated by the ultrasonic imaging method according to the first embodiment.
FIG. 7 is an example of the first intermediate composite image and the second intermediate composite image generated in the ultrasonic imaging method according to the second embodiment.

FIG. 8 is a schematic diagram for illustrating the weighted image synthesizing method performed by the ultrasonic imaging device according to the second embodiment.

FIG. 9 is a schematic diagram for illustrating the weighted image synthesizing method performed by the ultrasonic imaging device according to the second embodiment.

FIG. 10 is an example of the image obtained by visualizing the weighting matrix calculated according to various weighting rules applied in the ultrasonic imaging method according to the second embodiment.

FIG. 11 is a flowchart showing the processing procedure of the ultrasonic imaging method according to the second embodiment.

FIG. 12 is a flowchart showing the detailed processing procedure of step S6B in the second embodiment.

FIG. 13 is an example of the panorama composite image of the transverse section of the thigh, generated by the ultrasonic imaging method according to the second embodiment.

FIG. 14 is the ultrasonic images according to the conventional art, obtained by imaging a transverse section of the thigh in the linear scan mode and the sector scan mode, respectively.

DESCRIPTION OF EMBODIMENTS

Embodiments of the present invention will be described in detail hereinafter with reference to the accompanying drawings. In the following descriptions and drawings, the same reference numerals denote the same or similar components, and thus redundant descriptions of the same or similar components will be omitted.

[First embodiment]

(Overall configuration)

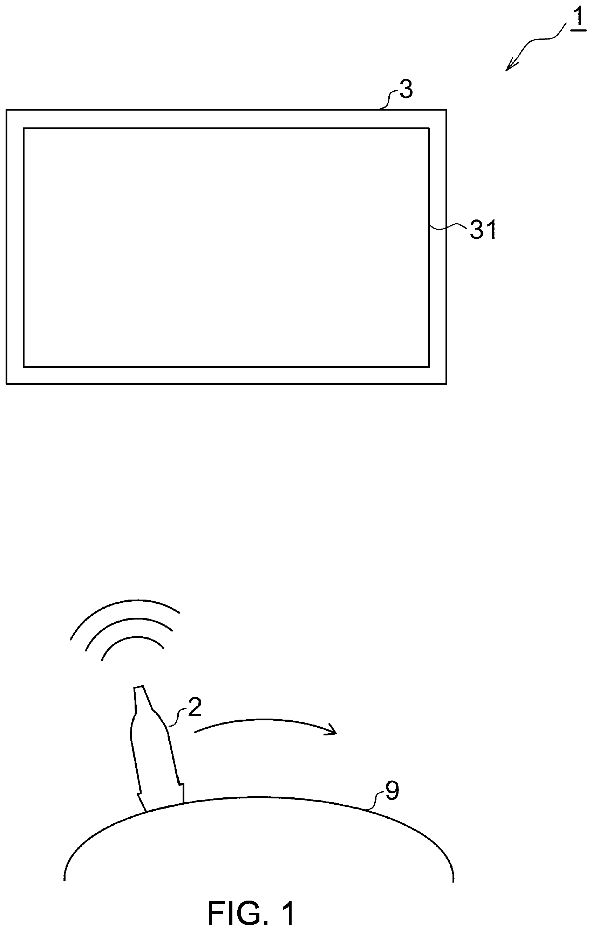

FIG. 1 is a schematic diagram showing the configuration of an ultrasonic imaging system 1. The ultrasonic imaging system 1 includes a probe 2 and an ultrasonic imaging device 3.

The probe 2 is a device that transmits ultrasonic waves from the surface of a specimen 9 toward the interior of the specimen 9 and receives the ultrasonic waves reflected in the interior of the specimen 9, and is configured to be held and moved by an examinee in this embodiment. The lower end of the probe 2 is provided with an ultrasonic transmission and reception surface on which a plurality of ultrasonic transducers are arranged in a row. In this embodiment, the specimen 9 is the thigh of a human body, but the body part included in the specimen 9 is not particularly limited.

In this embodiment, the probe 2 operates in two driving modes, which are a linear scan mode (corresponding to the first scan mode described in the claims) for acquiring a fragment image by linear scanning and a sector scan mode (corresponding to the second scan mode described in the claims) for acquiring a fragment image by sector scanning with a wider imaging range than linear scanning. The fragment image is an ultrasonic image obtained by performing one time of imaging in the linear scan mode or the sector scan mode, and is equivalent to an image captured by an ultrasonic diagnostic device (ultrasonic imaging device) having a general configuration.

When acquiring a panorama composite image for a transverse section of the specimen 9, the examinee brings the ultrasonic transmission and reception surface of the probe 2 into contact with the specimen 9 and moves the probe 2 along the surface of the specimen 9 (to scan around the thigh with the probe 2). In the meantime, the probe 2 intermittently transmits ultrasonic waves from the ultrasonic transmission and reception surface toward the interior of the specimen 9 while switching the scan mode between the linear scan mode and the sector scan mode at a particular cycle, and receives the ultrasonic waves reflected in the interior of the specimen 9 at the ultrasonic transmission and reception surface. Then, the probe 2 outputs electrical signals (echo signals) representing the received ultrasonic waves in each of the linear scan mode and the sector scan mode. Preferably, the probe 2 has an angle sensor attached thereto, and information about the inclination angle of the probe 2 (for example, inclination angle such as tilt of the probe 2 from the vertical direction) is transmitted to the ultrasonic imaging device 3 together with the echo signals.

The ultrasonic imaging device 3 is connected to the probe 2 in a wireless manner such as WiFi (registered trademark). In this embodiment, the ultrasonic imaging device 3 includes, for example, a tablet terminal, and has a function of generating a plurality of fragment images (a plurality of first fragment images and a plurality of second fragment images) in each of the linear scan mode and the sector scan mode based on the echo signals received from the probe 2, and furthermore displaying a panorama composite image of the transverse section, which is obtained by synthesizing these fragment images.

The ultrasonic imaging device 3 is not particularly limited as long as the ultrasonic imaging device 3 is capable of displaying images, and may include a general-purpose personal computer, a smart phone, etc. Besides, the method of connecting the probe 2 and the ultrasonic imaging device 3 is not particularly limited, and wired connection may also be used.

(Function of the ultrasonic imaging device)

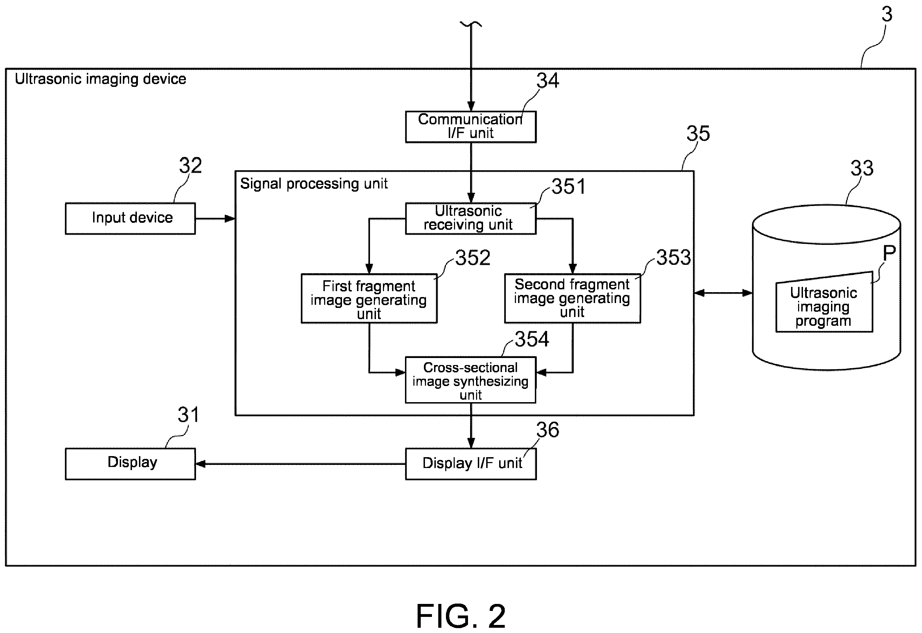

FIG. 2 is a block diagram showing the configuration of the ultrasonic imaging device 3. The ultrasonic imaging device 3 includes a display 31, an input device 32, an auxiliary storage device 33, a communication interface unit (I/F unit) 34, and a display interface unit (I/F unit) 36, as hardware configurations.

The display 31 may include, for example, a liquid crystal display, a plasma display, an organic EL (electroluminescence) display, etc. The display 31 may be configured as a device separate from the ultrasonic imaging device 3.

The input device 32 is a touch panel provided on the surface of the display 31. The examinee is allowed to perform an input operation on the image displayed on the display 31 via the input device 32.

The auxiliary storage device 33 is a non-volatile storage device that stores an operating system (OS), various control programs, data generated by the programs, etc., and includes, for example, an eMMC (embedded Multi Media Card), an SSD (Solid State Drive), etc. An ultrasonic imaging program P is stored in the auxiliary storage device 33. The ultrasonic imaging program P may be installed in the ultrasonic imaging device 3 via a network such as the Internet. Alternatively, the ultrasonic imaging program P may be installed in the ultrasonic imaging device 3 by causing the ultrasonic imaging device 3 to read a computer-readable non-temporary tangible recording medium such as an SD card or the like which records the ultrasonic imaging program P.

The communication interface unit 34 transmits and receives data to and from an external device, and performs demodulation of signals received from the probe 2, modulation of control signals to be transmitted to the probe 2, etc.

The display interface unit 36 develops various image data generated by the arithmetic processing of the ultrasonic imaging device 3 in a VRAM (Video Random Access Memory) to display the image on the display 31, and displays, for example, the composite image or the like generated by a signal processing unit 35, which will be described later, on the display 31.

Although not shown, the ultrasonic imaging device 3 further includes a processor such as a CPU which performs data processing, and a memory (main storage device) which the processor uses as a work area for data processing, as other hardware configurations.

The ultrasonic imaging device 3 also includes the signal processing unit 35 as a software configuration. The signal processing unit 35 is a functional block realized by the processor executing the ultrasonic imaging program P, and has a function of processing the echo signals received from the probe 2 and displaying the composite image of a transverse section of the specimen 9 on the display 31 for the examinee, doctor, imaging worker, etc. to easily grasp the state of the specimen 9. To realize this function, the signal processing unit 35 includes an ultrasonic receiving unit 351, a first fragment image generating unit 352, a second fragment image generating unit 353, and a cross-sectional image synthesizing unit 354. The signal processing unit 35 may be implemented in hardware by a logic circuit formed on an integrated circuit.

The ultrasonic receiving unit 351 generates a transmission signal by delaying a signal having a frequency in the ultrasonic range, and outputs the transmission signal to a control device (not shown) built in the probe 2. The control device drives the probe 2 based on the received transmission signal. The ultrasonic receiving unit 351 is capable of controlling the driving mode and beam shape of the probe 2 by controlling the delay. A reception signal is input from the probe 2 to the ultrasonic receiving unit 351. The ultrasonic receiving unit 351 performs processing such as analog-to-digital conversion on the input reception signal, and respectively outputs the processed reception signal to the first fragment image generating unit 352 during driving in the linear scan mode and to the second fragment image generating unit 353 during driving in the sector scan mode. While the probe 2 is moved along the surface of the specimen 9, the ultrasonic receiving unit 351 repeatedly outputs the transmission signal at regular time intervals for each of the linear scan mode and the sector scan mode, and acquires the reception signal of the ultrasonic waves received by the probe 2 each time the transmission signal is output.

The function of the ultrasonic receiving unit 351 may be provided in the control device that controls the probe 2. In that case, the control device may be connected to the ultrasonic imaging device 3, or the ultrasonic image may be stored in the control device and transmitted to the ultrasonic imaging device 3 via a recording medium.

The first fragment image generating unit 352 and the second fragment image generating unit 353 each generate fragment images, which are obtained by partially imaging the target, by performing an image conversion process corresponding to the driving mode of the probe 2 based on the reception signal output by the ultrasonic receiving unit 351. In this embodiment, the first fragment image generating unit 352 generates fragment images (first fragment images) in the linear scan mode, and the second fragment image generating unit 353 generates fragment images (second fragment images) in the sector scan mode. While the probe 2 is moved along the surface of the specimen 9, the first fragment image generating unit 352 and the second fragment image generating unit 353 each generate a plurality of fragment images (a plurality of first fragment images and a plurality of second fragment images) of the transverse section of the specimen 9 from various directions based on the reception signal repeatedly input from the ultrasonic receiving unit 351, together with angle information (information about inclination angle) of the probe 2 with respect to the surface of the specimen 9 at the time when the fragment images are acquired.

That is, in this embodiment, a pair of fragment images, namely, a first fragment image in the linear scan mode and a second fragment image in the sector scan mode, are generated at one inclination angle of the probe 2 with respect to the surface of the specimen 9, and while the probe 2 is continuously moved along the surface of the specimen 9, multiple pairs of such fragment images are generated for each inclination angle of the probe 2, together with information about the inclination angle of the probe 2. The number of pairs of fragment images generated varies depending on the transmission and reception time and transmission and reception cycle of the ultrasonic waves at the probe 2. For instance, one fragment image pair of the first fragment image and the second fragment image are generated about every 125 msec.

The cross-sectional image synthesizing unit 354 non-uniformly synthesizes the first fragment images generated by the first fragment image generating unit 352 and the second fragment images generated by the second fragment image generating unit 353. In this specification, the term "cross section" or "transverse section" is a concept including not only a circular section but also a partial section.

The composite image of the transverse section of the specimen 9 generated by the cross-sectional image synthesizing unit 354 is input to the display interface unit 36. The display interface unit 36 displays the composite image on the display 31 by developing data of the composite image in the VRAM.

Hereinafter, a form in which the cross-sectional image synthesizing unit 354 non-uniformly synthesizes the first fragment image and the second fragment image in the first embodiment will be described specifically.

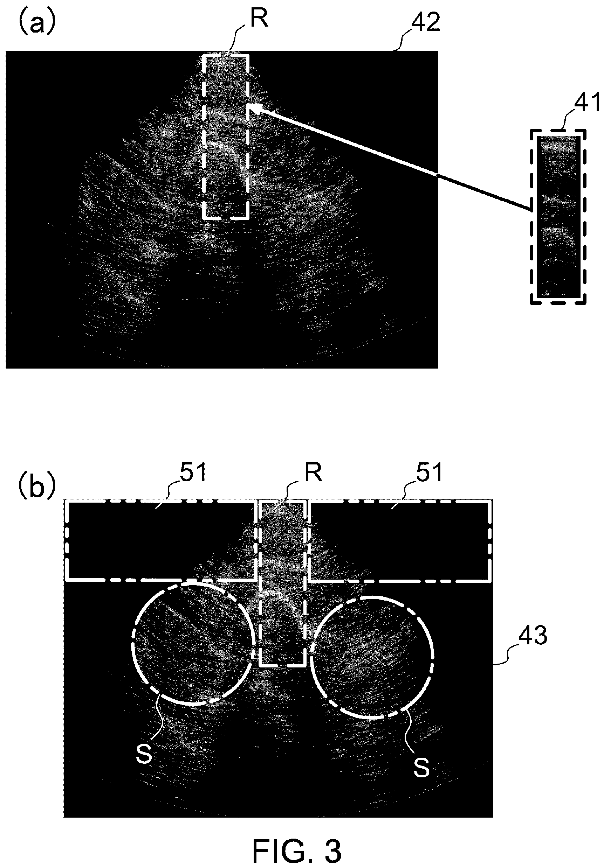

In the first embodiment, first, the process of non-uniformly synthesizing the first fragment image acquired in the linear scan mode and the second fragment image acquired in the sector scan mode is performed for each inclination angle of the probe 2 by the method described with reference to the schematic diagram of FIG. 3 later, to generate a plurality of intermediate composite images 43. Information about the inclination angle of the probe 2 is associated with the intermediate composite images 43.

Next, the plurality of intermediate composite images 43 generated for each inclination angle of the probe 2 are rotated based on the inclination angle of the probe 2 and synthesized, thereby generating a panorama composite image that captures the transverse section of the specimen 9 over a wide range.

(Synthesis of different types of fragment images)

FIG. 3 is a schematic diagram for illustrating the method of synthesizing different types of fragment images, performed by the ultrasonic imaging device according to the first embodiment.

(a) of FIG. 3 shows the first fragment image 41 and the second fragment image 42 before synthesis. In the first embodiment, as shown in (a) of FIG. 3, a region (region R surrounded by a dashed line), which corresponds to the first fragment image 41, in the second fragment image 42 acquired in the sector scan mode is replaced with the first fragment image 41 acquired in the linear scan mode so as to partially superimpose and synthesize the first fragment image 41 on the second fragment image 42 and generate the intermediate composite image 43.
(b) of FIG. 3 shows the intermediate composite image 43 at an angle of the probe 2. In the intermediate composite image 43, the first fragment image 41 is arranged in the foreground in the region R surrounded by the dashed line, and the interior of the muscle is shown clearly. In addition, the second fragment image 42 remains in a region S surrounded by a one-dot chain line, and the septum is shown clearly.

Thus, the above method is capable of generating a clear composite image of the transverse section of the thigh, which takes advantages of the merits of both the linear scan mode and the sector scan mode, for the transverse section of the thigh, which is the specimen 9.

Image processing for noise removal may be further performed on regions 51 that are surrounded by two-dot chain lines and located on both sides of the region R surrounded by the dashed line in the intermediate composite image 43 shown in (b) of FIG. 3, thereby further improving the clarity of the panorama composite image generated in the subsequent processes.

(Processing procedure)

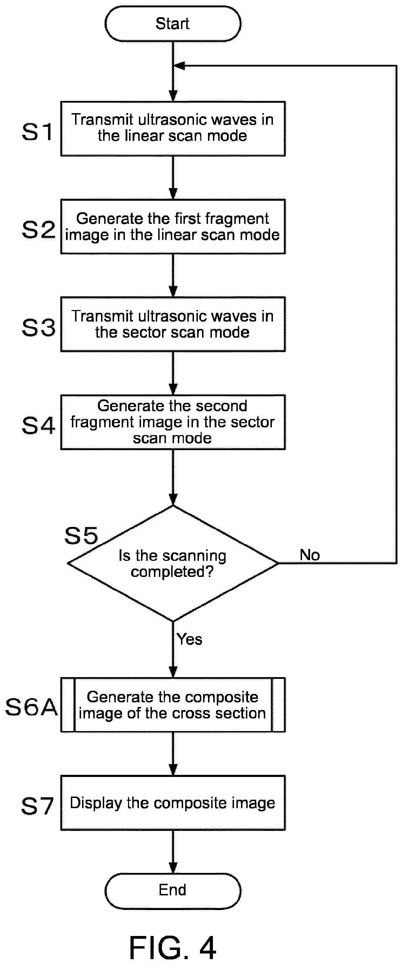

FIG. 4 is a flowchart showing the processing procedure of an ultrasonic imaging method according to the first embodiment.

In step S1, the ultrasonic receiving unit 351 (control device) drives the probe 2 in the linear scan mode, and the probe 2 transmits ultrasonic waves from the surface of the specimen 9 toward the interior of the specimen 9 in the linear scan mode. Then, the probe 2 receives the ultrasonic waves reflected in the interior of the specimen 9, and an echo signal corresponding to the linear scan mode is output from the probe 2.

In step S2, the ultrasonic receiving unit 351 performs processing such as analog-to-digital conversion on the input reception signal, and outputs the processed reception signal to the first fragment image generating unit 352. The first fragment image generating unit 352 generates the first fragment image 41 in the linear scan mode. In this embodiment, the first fragment image generating unit 352 generates the first fragment image 41 each time the probe 2 outputs an echo signal.

In step S3, similar to step S1, the ultrasonic receiving unit 351 drives the probe 2 in the sector scan mode. An echo signal corresponding to the sector scan mode is output from the probe 2.

In step S4, similar to step S2, the second fragment image generating unit 353 generates the second fragment image 42 in the sector scan mode each time the probe 2 outputs an echo signal.

Steps S1 to S4 are repeated until the scanning of the probe 2 is completed. As a result, a plurality of first fragment images 41 corresponding to a plurality of mutually different positions on the surface of the specimen 9 are generated for each inclination angle of the probe 2. Similarly, a plurality of second fragment images 42 corresponding to a plurality of mutually different positions on the surface of the specimen 9 are generated for each inclination angle of the probe 2.

When the scanning of the probe 2 is completed (Yes in step S5), in step S6A (image synthesizing step), the cross-sectional image synthesizing unit 354 generates a composite image of a cross section of the specimen 9 by non-uniformly synthesizing the plurality of first fragment images and the plurality of second fragment images.

Thereafter, in step S7 (display step), a panorama composite image of the transverse section of the specimen 9 is displayed on the display 31.

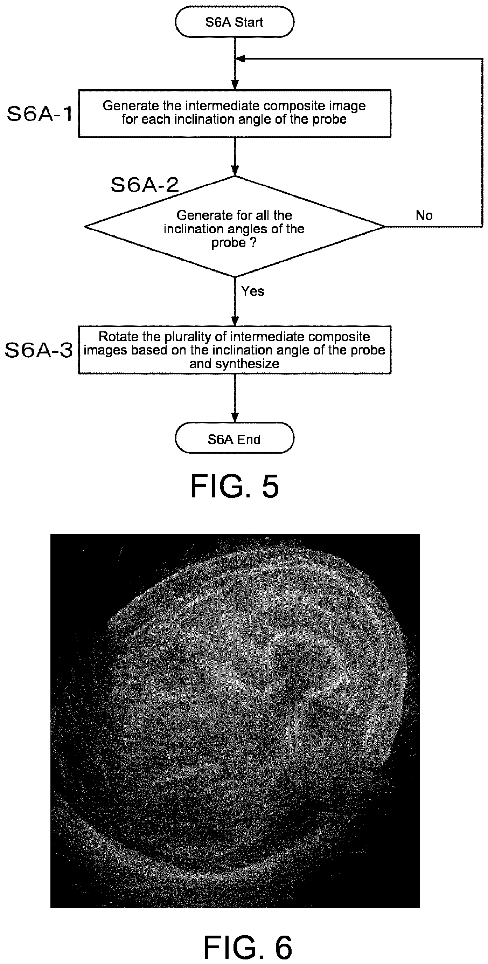

FIG. 5 is a flowchart showing the detailed processing procedure of step S6A in the first embodiment. Step S6A has steps S6A-1 to S6A-3.

First, in step S6A-1, the cross-sectional image synthesizing unit 354 generates the intermediate composite image 43 for each inclination angle of the probe 2. The cross-sectional image synthesizing unit 354 partially superimposes and synthesizes the first fragment image 41 on the second fragment image 42 by the synthesizing method described with reference to FIG. 3, so as to generate the intermediate composite image 43.

Step S6A-1 is repeated until generation of the intermediate composite image 43 is completed for all the inclination angles of the probe (Yes in step S6A-2). Thereby, a plurality of intermediate composite images 43 are generated for each inclination angle of the probe 2.

Subsequently, in step S6A-3, the cross-sectional image synthesizing unit 354 rotates the plurality of intermediate composite images 43 based on the inclination angle of the probe 2 and synthesizes the intermediate composite images 43. Thereby, a panorama composite image of the transverse section of the specimen 9 is generated. FIG. 6 illustrates an example of the panorama composite image of the transverse section of the thigh, generated by the ultrasonic imaging method according to the first embodiment.

When synthesizing the plurality of intermediate composite images 43, the cross-sectional image synthesizing unit 354 is capable of detecting and matching feature amounts between regions respectively included in the plurality of intermediate composite images 43 to determine the position for superimposing the plurality of intermediate composite images 43. At this time, preferably, the cross-sectional image synthesizing unit 354 rotates the intermediate composite image 43 based on the detected angle obtained from the angle sensor of the probe 2, and performs the matching based on the rotated intermediate composite image 43, so as to accurately correct the rotation angle of each intermediate composite image 43 and determine the position for superimposing the intermediate composite images 43 with higher accuracy.

A well-known technique can be applied to the process of rotating and synthesizing the plurality of intermediate composite images 43 to generate one panorama composite image. In this embodiment, for example, the plurality of intermediate composite images 43 are synthesized using feature point matching between the intermediate composite images 43.

In this method, feature points are detected from the first intermediate composite image and the second intermediate composite image. Then, the feature points of the first intermediate composite image and the second intermediate composite image are matched to calculate a homogeneous transformation matrix of the first intermediate composite image and the second intermediate composite image. Specifically, when the second intermediate composite image is rotated clockwise by $\theta$ and translated by $t_x$ in the x-axis direction and by $t_y$ in the y-axis direction with respect to the first intermediate composite image, in the case where the feature points of the first intermediate composite image and the second intermediate composite image match, the homogeneous transformation matrix R for moving the coordinate system of the second intermediate composite image to align with the first intermediate composite image is:

[Formula 1]

$$R = \begin{bmatrix} cos\theta & -sin\theta & t_x \\ sin\theta & cos\theta & t_y \\ 0 & 0 & 1 \end{bmatrix}.$$

That is, when the feature point (x, y) on the first intermediate composite image moves to the feature point (x', y') on the second intermediate composite image, the relationship of:

[Formula 2]

$$\begin{bmatrix} x' \\ y' \\ 1 \end{bmatrix} = R \begin{bmatrix} x \\ y \\ 1 \end{bmatrix}$$

is established. Since the coordinates of the feature point contain errors, and the correspondence relationship itself determined due to the influence of noise contains errors, outliers that adversely affect the calculation are excluded by the RANSAC (RANdom SAmple Consensus) algorithm. Moreover, non-linear least-squares methods such as the Gauss-Newton method and the Levenberg-Marquardt method can be used to calculate the positional relationship.

The homogeneous transformation matrix R is sequentially calculated for two intermediate composite images that are adjacent in generation order, and the calculation is performed up to the n-1th intermediate composite image and the nth intermediate composite image. When the homogeneous transformation matrix from the k+1th ($1 \leq k \leq n-1$) intermediate composite image to the kth intermediate composite image is $R_k$, the homogeneous transformation matrix from the k+1th intermediate composite image to the first intermediate composite image is $R_1 R_2 ... R_k$. The coordinate system of the first intermediate composite image is called the world coordinate system, and the coordinates of all the intermediate composite images can be calculated by calculating the homogeneous transformation matrices to the world coordinate system for all the intermediate composite images. Then, a single panorama composite image is generated by blending the pixels of all the intermediate composite images.

(Summary)

As described above, in the first embodiment, the first fragment image acquired in the linear scan mode and the second fragment image acquired in the sector scan mode are synthesized non-uniformly, so as to generate a clear composite image of the transverse section of a human body, which takes advantages of the merits of both the linear scan mode and the sector scan mode, for the transverse section of the human body, which is the specimen 9.

[Second embodiment]

Hereinafter, a form in which the cross-sectional image synthesizing unit 354 non-uniformly synthesizes the first fragment image and the second fragment image in the second embodiment will be described specifically.

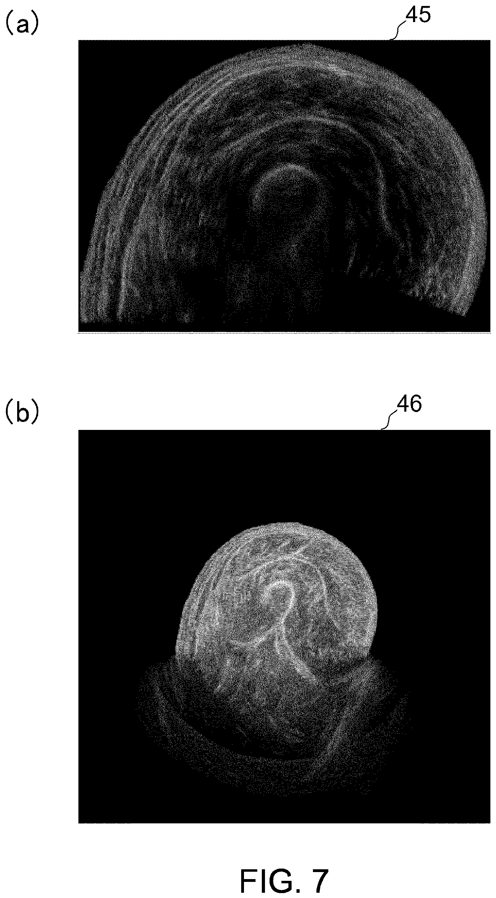

In the second embodiment, first, a plurality of first fragment images acquired in the linear scan mode, which are generated for each inclination angle of the probe 2, are rotated based on the inclination angle of the probe 2 and synthesized to generate a first intermediate composite image 45. Similarly for the second fragment image acquired in the sector scan mode, a plurality of second fragment images generated for each inclination angle of the probe 2 are rotated based on the inclination angle of the probe 2 and synthesized to generate a second intermediate composite image 46. An example of the first intermediate composite image 45 and the second intermediate composite image 46 generated by the ultrasonic imaging method according to the second embodiment is illustrated in (a) and (b) of FIG. 7. Both the first intermediate composite image 45 and the second intermediate composite image 46 are panorama composite images obtained by imaging a transverse section of the specimen 9 over a wide range.

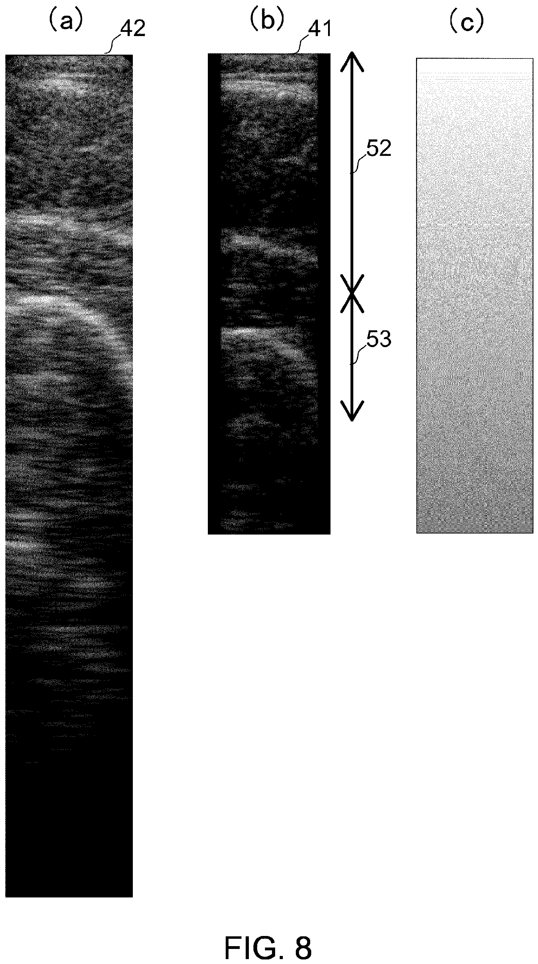

Next, the first intermediate composite image 45 and the second intermediate composite image 46 are synthesized with weighting by the method described with reference to the schematic diagrams of FIG. 8 to FIG. 10, which will be described later, to generate a panorama composite image in which the first fragment image and the second fragment image are non-uniformly synthesized.

(Synthesis of the same type of fragment images)

The cross-sectional image synthesizing unit 354 rotates a plurality of first fragment images generated for each inclination angle of the probe 2 based on the inclination angle of the probe 2 and synthesizes the plurality of first fragment images to generate the first intermediate composite image 45. Similarly, the cross-sectional image synthesizing unit 354 rotates a plurality of second fragment images generated for each inclination angle of the probe 2 based on the inclination angle of the probe 2 and synthesizes the plurality of second fragment images to generate the second intermediate composite image 46.

When generating the first intermediate composite image 45 and the second intermediate composite image 46 respectively, the cross-sectional image synthesizing unit 354 is capable of detecting and matching feature amounts between regions respectively included in the plurality of fragment images to determine the position for superimposing the plurality of fragment images. At this time, preferably, the cross-sectional image synthesizing unit 354 rotates the fragment image based on the detected angle obtained from the angle sensor of the probe 2, and performs the matching based on the rotated fragment image, so as to accurately correct the rotation angle of each fragment image and determine the position for superimposing the fragment image with higher accuracy.

A well-known technique can be applied to the process of rotating and synthesizing the plurality of fragment images to generate one intermediate composite image. In this embodiment, for example, the plurality of fragment images are synthesized using feature point matching between the fragment images. The method for feature point matching has been described in the first embodiment above with the intermediate composite image as an example, so detailed description thereof will be omitted in this embodiment.

(Weighted synthesis)

FIG. 8 to FIG. 10 are schematic diagrams for illustrating the weighted image synthesizing method performed by the ultrasonic imaging device according to the second embodiment.

In the second embodiment, a weighting rule is applied to the intermediate composite images 45 and 46 respectively generated for the linear scan mode and the sector scan mode to synthesize the intermediate composite images 45 and 46, thereby generating a clear composite image of the transverse section of the human body, which takes advantages of the merits of both the linear scan mode and the sector scan mode, for the transverse section of the human body, which is the specimen 9. The weighting rule can use, for example, both or at least one of weighting according to depth and weighting according to inclination angle, which will be exemplified below. When weighting according to depth and weighting according to inclination angle are both applied, preferably, weighting according to inclination angle is applied more strongly than weighting according to depth.

FIG. 8 is a schematic diagram for illustrating weighting according to depth, (a) of FIG. 8 shows an image obtained by cutting out the second fragment image 42 acquired in the sector scan mode from the surface of the specimen 9 in the depth direction. (b) of FIG. 8 shows the first fragment image 41 acquired in the linear scan mode. The upward direction in the drawing corresponds to a region that is shallow from the surface in the interior of the specimen 9, and the downward direction corresponds to a region that is deep from the surface.

In weighting according to depth, the first intermediate composite image 45 and the second intermediate composite image 46 are synthesized with weighting that increases the ratio of the first intermediate composite image 45 in the linear scan mode for the region shallow from the surface in the interior of the specimen 9 and increases the ratio of the second intermediate composite image 46 in the sector scan mode for the region deep from the surface.

One of the reasons for increasing the ratio of contribution of the linear scan mode for the region shallow from the surface is that, as indicated by reference numeral 52 in the fragment image of (b) of FIG. 8, the influence of noise is small in the shallow region, and the interior of the muscle is clearly imaged. One of the reasons for increasing the ratio of contribution of the sector scan mode for the region deep from the surface is that, as indicated by reference numeral 53 in the fragment image of (b) of FIG. 8, ultrasonic echoes are attenuated in the linear scan mode in the deep region, (c) of FIG. 8 illustrates an image visualizing an example of a weighting matrix calculated according to such a weighting rule.

In the image of (c) of FIG. 8, white means that weighting of the linear scan mode is strong, black means that weighting of the sector scan mode is strong, and multiple shades of gray mean weighting in between. It can be seen that the degree of black gradually increases from the shallow region to the deep region, and the weighting of the sector scan mode gradually increases.

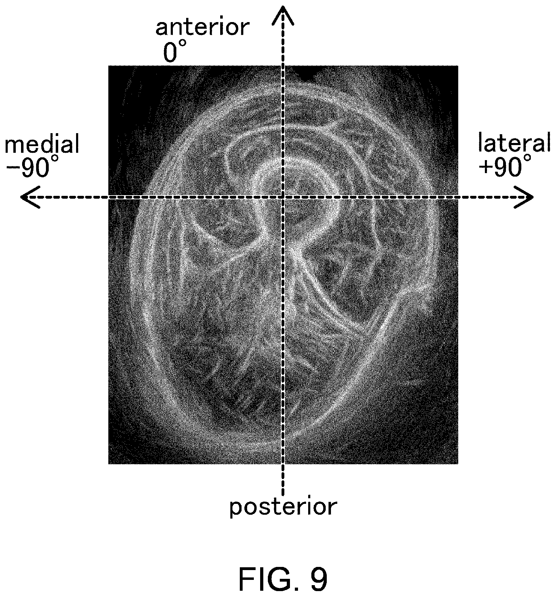

FIG. 9 is a schematic diagram for illustrating weighting according to the inclination angle of the probe, and shows an example of the correspondence relationship between the inclination angle of the probe 2 and the thigh. The composite image of the transverse section of the thigh used to exemplify the correspondence relationship is a composite image obtained only in the sector scan mode according to the conventional art.

For convenience, assuming that the thigh of the human body is measured in a sitting or supine position, the anterior of the thigh corresponds to the inclination angle of the probe 2 near 0 degree. Similarly, the medial and lateral sides of the thigh correspond to inclination angles of the probe 2 near ±90 degrees.

In weighting according to the inclination angle of the probe, the first intermediate composite image 45 and the second intermediate composite image 46 are synthesized with weighting that increases the ratio of the first intermediate composite image 45 in the linear scan mode for the inclination angle near 0 degree and increases the ratio of the second intermediate composite image 46 in the sector scan mode for the inclination angles near ±90 degrees.

One of the reasons for increasing the ratio of contribution of the linear scan mode for the inclination angle near 0 degree is that the septum is less likely to be included in the ultrasonic image captured at the inclination angle near 0 degree. One of the reasons for increasing the ratio of contribution of the sector scan mode for the inclination angles near ±90 degrees is to include the septum in the captured ultrasonic image. This is because, at the inclination angles near ±90 degrees, the outline of the septum and the ultrasonic waves emitted from the probe 2 gradually become substantially parallel, and in the linear scan mode, the ultrasonic waves reflected by the septum cannot be received clearly.

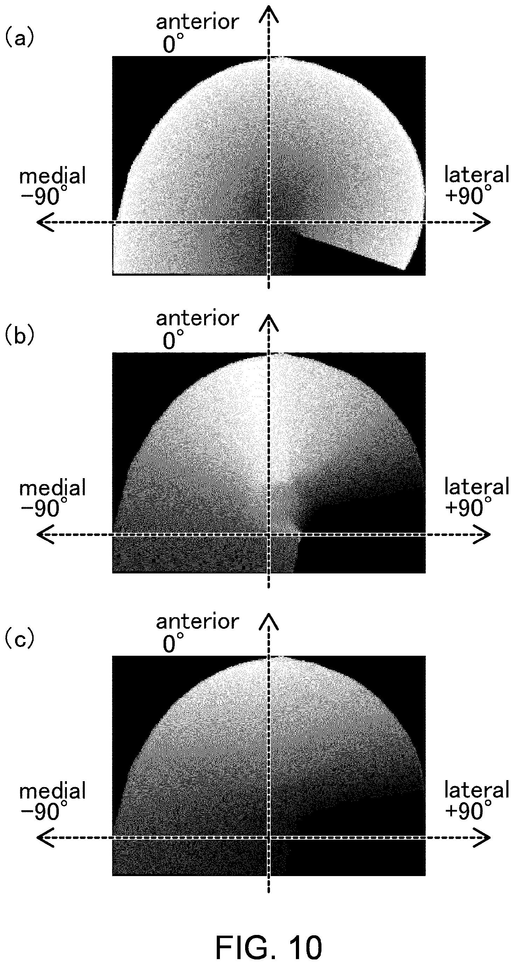

FIG. 10 illustrates an example of the image visualizing the weighting matrix calculated according to such a weighting rule. (a) of FIG. 10 is an image visualizing the weighting matrix that considers only the depth, and (b) of FIG. 10 is an image visualizing the weighting matrix that considers only the inclination angle of the probe. (c) of FIG. 10 is an image visualizing the weighting matrix that considers both the depth and the inclination angle.

In the images shown in FIG. 10, white means that weighting of the linear scan mode is strong, black means that weighting of the sector scan mode is strong, and multiple shades of gray mean weighting in between. Referring to the image of (a) of FIG. 10, for example, it can be seen that the shade does not change at the same depth even when the inclination angle changes from near 0 degree to near ±90 degrees. Further, referring to the image of (b) of FIG. 10, for example, it can be seen that the degree of blackness gradually increases as the inclination angle approaches ±90 degrees from near 0 degree, and the weighting of the sector scan mode gradually increases.

(Processing procedure)

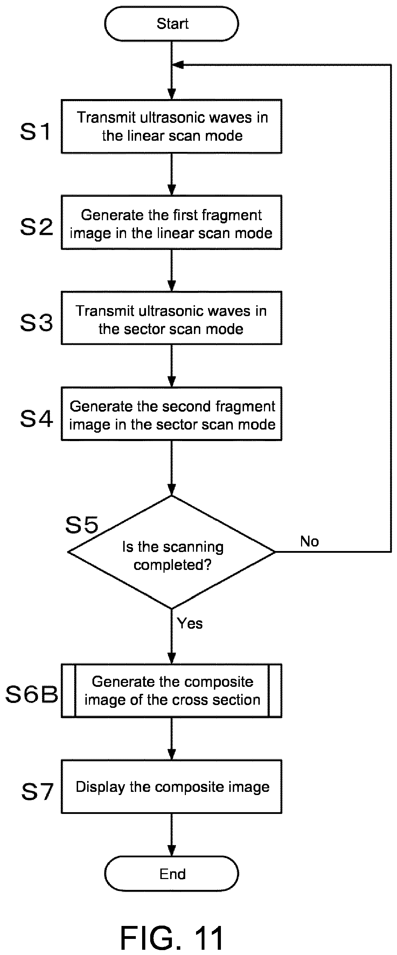

FIG. 11 is a flowchart showing the processing procedure of the ultrasonic imaging method according to the second embodiment. The overall processing procedure in the flowchart shown in FIG. 11 is the same as the flowchart shown in FIG. 4, but step S6A for generating a composite image of the cross section is replaced with step S6B.

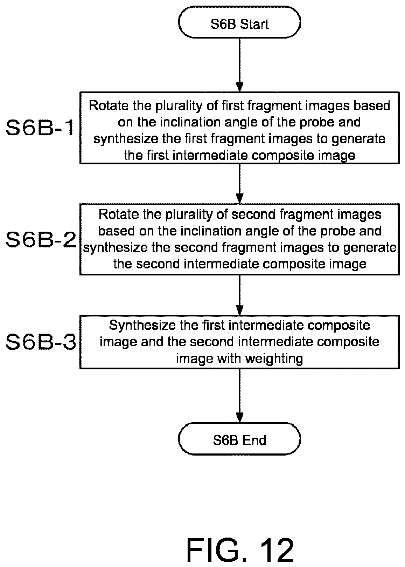

FIG. 12 is a flowchart showing the detailed processing procedure of step S6B in the second embodiment. Step S6B has steps S6B-1 to S6B-3.

First, in step S6B-1, the cross-sectional image synthesizing unit 354 rotates a plurality of first fragment images 41 acquired in the linear scan mode based on the inclination angle of the probe 2 and synthesizes the first fragment images 41 to generate a first intermediate composite image 45, which is synthesized using only fragment images related to the linear scan mode. At this point, the first intermediate composite image 45 is a panorama composite image that captures the transverse section of the specimen 9 over a wide range.

In step S6B-2, similar to step S6B-1, the cross-sectional image synthesizing unit 354 rotates a plurality of second fragment images 42 acquired in the sector scan mode based on the inclination angle of the probe 2 and synthesizes the second fragment images 42 to generate a second intermediate composite image 46, which is synthesized using only fragment images related to the sector scan mode. At this point, the second intermediate composite image 46 is also a panorama composite image that captures the transverse section of the specimen 9 over a wide range.

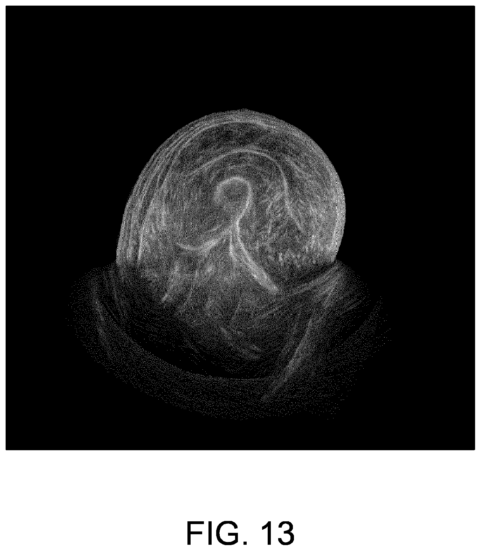

Subsequently, in step S6B-3, the cross-sectional image synthesizing unit 354 synthesizes the first intermediate composite image 45 and the second intermediate composite image 46 with weighting by the weighted synthesizing method described with reference to FIG. 8 to FIG. 10. In this embodiment, both weighting according to depth and weighting according to inclination angle, as illustrated in (c) of FIG. 10, are applied as the weighting rule. As a result, a panorama composite image of the transverse section of the specimen 9, in which the first fragment image and the second fragment image are non-uniformly synthesized, is generated. FIG. 13 illustrates an example of the panorama composite image of the transverse section of the thigh, generated by the ultrasonic imaging method according to the second embodiment.

(Summary)

As described above, in the second embodiment, the intermediate composite images respectively generated for the linear scan mode and the sector scan mode are synthesized by applying the weighting rule thereto, so as to generate a clear composite image of the transverse section of a human body, which takes advantages of the merits of both the linear scan mode and the sector scan mode, for the transverse section of the human body, which is the specimen 9.

[Other forms]

Although the present invention has been described based on specific embodiments, the present invention is not limited to the embodiments described above.

In the above embodiment, to obtain fragment images corresponding to a plurality of mutually different positions on the surface of the specimen 9, the probe 2 intermittently transmits ultrasonic waves while the probe 2 is moved along the surface of the specimen 9. However, the mode for obtaining the fragment images is not limited thereto. For example, a plurality of probes 2 may be arranged on the specimen 9, and ultrasonic waves may be transmitted simultaneously from the probes 2.

In the above embodiment, the probe 2 operates in both the driving modes, namely, the linear scan mode and the sector scan mode, but the driving modes of the probe 2 are not limited thereto. The probe 2 may operate in a convex scan mode instead of the sector scan mode. That is to say, the probe 2 may operate in both the linear scan mode and the convex scan mode.

In the first embodiment described above, in step S6A-1, the cross-sectional image synthesizing unit 354 partially superimposes and synthesizes the first fragment image 41 on the second fragment image 42 by the synthesizing method described with reference to FIG. 3 to generate the intermediate composite image 43. However, the mode for generating the intermediate composite image 43 is not limited thereto. The weighting rule described with reference to FIG. 8 to FIG. 10 may be applied when the cross-sectional image synthesizing unit 354 partially superimposes and synthesizes the first fragment image 41 on the second fragment image 42.

In the first embodiment described above, in step S6A-3, the cross-sectional image synthesizing unit 354 rotates the plurality of intermediate composite images 43 based on the inclination angle of the probe 2 and synthesizes the intermediate composite images 43 to generate the panorama composite image of the transverse section of the specimen 9. However, the mode for generating the panorama composite image is not limited thereto. The weighting rule described with reference to FIG. 8 to FIG. 10 may be applied when the cross-sectional image synthesizing unit 354 rotates the plurality of intermediate composite images 43 based on the inclination angle of the probe 2 and synthesizes the intermediate composite images 43.

Industrial applicability

The present invention is applicable to both medical and non-medical purposes, and is particularly suitable for use by an examinee who is not a medical professional to visualize and routinely check his/her own muscle conditions.

Reference Signs List

1 ultrasonic imaging system
2 probe
3 ultrasonic imaging device
9 specimen
31 display
32 input device
33 auxiliary storage device
34 communication interface unit
35 signal processing unit
36 display interface unit
41 first fragment image
42 second fragment image
43 intermediate composite image
45 first intermediate composite image
46 second intermediate composite image
351 ultrasonic receiving unit
352 first fragment image generating unit
353 second fragment image generating unit
354 cross-sectional image synthesizing unit

**Claims**

1. An ultrasonic imaging device, comprising:

an ultrasonic receiving unit configured to receive ultrasonic waves that are transmitted in two scan modes, which are a first scan mode and a second scan mode having a wider imaging range than the first scan mode, from a probe arranged on a surface of a specimen toward an interior of the specimen and that are reflected in the interior of the specimen with the probe;
a first fragment image generating unit configured to generate a first fragment image obtained by partially imaging the interior of the specimen in the first scan mode based on the ultrasonic waves;
a second fragment image generating unit configured to generate a second fragment image obtained by partially imaging the interior of the specimen in the second scan mode based on the ultrasonic waves; and
a cross-sectional image synthesizing unit configured to generate a composite image of a cross section of the specimen by non-uniformly synthesizing the first fragment image and the second fragment image.

2. The ultrasonic imaging device according to claim 1, wherein the cross-sectional image synthesizing unit is further configured to generate a plurality of intermediate composite images by replacing a region of the second fragment image, which corresponds to the first fragment image, with the first fragment image for each inclination angle of the probe, and
generate the composite image of the cross section of the specimen by synthesizing the plurality of intermediate composite images generated for each inclination angle of the probe based on the inclination angle of the probe.

3. The ultrasonic imaging device according to claim 2, wherein the cross-sectional image synthesizing unit is further configured to generate the plurality of intermediate composite images for each inclination angle of the probe by synthesizing the first fragment image and the second fragment image by weighted synthesis based on at least one of weighting based on a depth from the surface of the specimen and weighting based on the inclination angle of the probe when generating the plurality of intermediate composite images by replacing the region of the second fragment image, which corresponds to the first fragment image, with the first fragment image for each inclination angle of the probe.

4. The ultrasonic imaging device according to claim 2 or 3, wherein the cross-sectional image synthesizing unit is further configured to generate the composite image of the cross section of the specimen by synthesizing the plurality of intermediate composite images by weighted synthesis based on at least one of weighting based on a depth from the surface of the specimen and weighting based on the inclination angle of the probe when synthesizing, based on the inclination angle of the probe, the plurality of intermediate composite images generated by replacing the region of the second fragment image, which corresponds to the first fragment image, with the first fragment image for each inclination angle of the probe.

5. The ultrasonic imaging device according to claim 1, wherein the cross-sectional image synthesizing unit is further configured to generate a first intermediate composite image by synthesizing a plurality of the first fragment images generated for each inclination angle of the probe based on the inclination angle of the probe,

generate a second intermediate composite image by synthesizing a plurality of the second fragment images generated for each inclination angle of the probe based on the inclination angle of the probe, and
generate the composite image of the cross section of the specimen by synthesizing the first intermediate composite image and the second intermediate composite image by weighted synthesis based on at least one of weighting based on a depth from the surface of the specimen and weighting based on the inclination angle of the probe.

6. The ultrasonic imaging device according to any one of claims 1 to 5, wherein the first scan mode is a linear scan mode, and
the second scan mode is a sector scan mode or a convex scan mode.

7. An ultrasonic imaging method, comprising:

an ultrasonic reception step of receiving ultrasonic waves that are transmitted in two scan modes, which are a first scan mode and a second scan mode having a wider imaging range than the first scan mode, from a probe arranged on a surface of a specimen toward an interior of the specimen and that are reflected in the interior of the specimen with the probe;
a first fragment image generation step of generating a first fragment image obtained by partially imaging the interior of the specimen in the first scan mode based on the ultrasonic waves;
a second fragment image generation step of generating a second fragment image obtained by partially imaging the interior of the specimen in the second scan mode based on the ultrasonic waves; and
a cross-sectional image synthesizing step of generating a composite image of a cross section of the specimen by non-uniformly synthesizing the first fragment image and the second fragment image.

8. An ultrasonic imaging system, comprising:

a probe configured to transmit ultrasonic waves from a surface toward an interior of a specimen and receive the ultrasonic waves that are reflected in the interior of the specimen; and
the ultrasonic imaging device according to any one of claims 1 to 6.

9. An ultrasonic imaging program, enabling a computer to operate as:

an ultrasonic receiving unit receiving ultrasonic waves that are transmitted in two scan modes, which are a first scan mode and a second scan mode having a wider imaging range than the first scan mode, from a probe arranged on a surface of a specimen toward an interior of the specimen and that are reflected in the interior of the specimen with the probe;
a first fragment image generating unit generating a first fragment image obtained by partially imaging the interior of the specimen in the first scan mode based on the ultrasonic waves;
a second fragment image generating unit generating a second fragment image obtained by partially imaging the interior of the specimen in the second scan mode based on the ultrasonic waves; and
a cross-sectional image synthesizing unit generating a composite image of a cross section of the specimen by non-uniformly synthesizing the first fragment image and the second fragment image.

FIG. 1

FIG. 2

FIG. 3

**FIG. 4**

S6A Start

S6A-1 | Generate the intermediate composite image for each inclination angle of the probe

S6A-2 — Generate for all the inclination angles of the probe ?

No

Yes

S6A-3 | Rotate the plurality of intermediate composite images based on the inclination angle of the probe and synthesize

S6A End

## FIG. 5

## FIG. 6

(a)

45

(b)

46

FIG. 7

FIG. 8

FIG. 9

(a)

anterior
0°

medial
−90°

lateral
+90°

(b)

anterior
0°

medial
−90°

lateral
+90°

(c)

anterior
0°

medial
−90°

lateral
+90°

FIG. 10

Start

S1 | Transmit ultrasonic waves in the linear scan mode

S2 | Generate the first fragment image in the linear scan mode

S3 | Transmit ultrasonic waves in the sector scan mode

S4 | Generate the second fragment image in the sector scan mode

S5 | Is the scanning completed?

No

Yes

S6B | Generate the composite image of the cross section

S7 | Display the composite image

End

## FIG. 11

S6B Start

S6B-1 | Rotate the plurality of first fragment images based on the inclination angle of the probe and synthesize the first fragment images to generate the first intermediate composite image

S6B-2 | Rotate the plurality of second fragment images based on the inclination angle of the probe and synthesize the second fragment images to generate the second intermediate composite image

S6B-3 | Synthesize the first intermediate composite image and the second intermediate composite image with weighting

S6B End

# FIG. 12

FIG. 13

FIG. 14

## INTERNATIONAL SEARCH REPORT

| International application No. |
|---|
| **PCT/JP2022/009077** |

**A. CLASSIFICATION OF SUBJECT MATTER**

***A61B 8/14***(2006.01)i
FI:   A61B8/14

According to International Patent Classification (IPC) or to both national classification and IPC

**B. FIELDS SEARCHED**

Minimum documentation searched (classification system followed by classification symbols)

A61B8/00-8/15

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2017/010193 A1 (FURUNO ELECTRIC CO) 19 January 2017 (2017-01-19) paragraphs [0039]-[0062], fig. 1-8 | 1, 6-9 |
| A | | 2-5 |
| A | WO 2018/135188 A1 (FURUNO ELECTRIC CO) 26 July 2018 (2018-07-26) paragraph [0091], fig. 16 | 1-9 |
| A | WO 2020/039796 A1 (FURUNO ELECTRIC CO) 27 February 2020 (2020-02-27) paragraph [0035], fig. 7 | 1-9 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 May 2022** | **24 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/009077**

| Patent document cited in search report | | | Publication date (day/month/year) | Patent family member(s) | | | Publication date (day/month/year) |
|---|---|---|---|---|---|---|---|
| WO | 2017/010193 | A1 | 19 January 2017 | US | 2018/0263596 | A1 | |
| | | | | paragraphs [0049]-[0072], fig. 1-8 | | | |
| | | | | EP | 3323352 | A1 | |
| | | | | CN | 107835663 | A | |
| WO | 2018/135188 | A1 | 26 July 2018 | US | 2019/0365358 | A1 | |
| | | | | paragraph [0110], fig. 16 | | | |
| | | | | EP | 3572000 | A1 | |
| | | | | CN | 110114001 | A | |
| WO | 2020/039796 | A1 | 27 February 2020 | US | 2021/0169454 | A1 | |
| | | | | paragraph [0053], fig. 7 | | | |
| | | | | EP | 3841983 | A1 | |
| | | | | CN | 112367922 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017010193 A